# EUROPEAN PATENT SPECIFICATION

(11) **EP 2 070 531 B1**
(45) Date of publication and mention of the grant of the patent: **07.08.2019**
(21) Application number: 07425779.1
(22) Date of filing: 10.12.2007
(51) Int. Cl.: A61K 31/34, A61P 17/02

(54) **Pharmaceutical preparation containing histamine H2-receptor antagonists having wound healing activity at low doses**
Pharmazeutische Zubereitung mit Histamin-H2-Rezeptorantagonisten mit wundheilender Wirkung bei geringen Dosen
Préparation pharmaceutique contenant des antagonistes de récepteur H2 d'histamine ayant une activité cicatrisante à faibles doses

(43) Date of publication of application: 17.06.2009
(73) Proprietor: ITALFAR S.r.l., 00071 Pomezia, (RM) (IT)
(72) Inventor: Russo, Domenico, 89100 Reggio Calabria (IT)
(74) Representative: Long, Giorgio

(56) References cited:
- WO-A-92/02218
- DATABASE MEDLINE [Online] US NATIONAL LIBRARY OF MEDICINE (NLM), BETHESDA, MD, US; April 1981 (1981-04), DABROWSKI R ET AL: "The role of histamine in wound healing. II. The effect of antagonists and agonists of histamine receptors (H1 and H2) on collagen levels in granulation tissue." XP002479385 Database accession no. NLM6113740 & AGENTS AND ACTIONS APR 1981, vol. 11, no. 1-2, April 1981 (1981-04), pages 122-124, ISSN: 0065-4299

## Description

The present invention relates to a pharmaceutical preparation containing low doses of one or more histamine receptor antagonists according to claim 1 for use in the topic treatment of dermatologic, vaginal, rectal, and ophthalmic wounds, abrasions, ulcerations, burns, and sores.

A wound is a traumatic event of the skin, that is a continuous injury of the body soft structures produced by an injuring agent in several fashions.

Wound healing is a complex, dynamic biological process in which a sequence of events initiates leading to the repair of the wounds. Duration and end result vary according to the wound characteristic.

In particular, a blood clot is formed in the wound which is characterized by a network of fibrin which contains red blood cells, white blood cells, platelets, and other blood components.

Subsequently, other blood elements produce macrophages, which are cells with lysis ability, therefore able to clear fibrin and cell debris from the wound.

After the initial 24 - 48 hours, a so-called granulation tissue is created, which is composed by some cellular elements called fibroblasts, which penetrate the wound by following the fibres of the network of fibrin consisting in fibrin and myofibrils with elastic ability.

At the same time, vascular and then lymphatic sprouts are formed on the wound edges which extend to the center until meeting the sprouts coming from the opposite side, which they anastomize with to originate, as soon as they become canalized, a new blood and lymphatic network.

The fibroblasts have also the function of secreting a substance called hyaluronic acid, which is able to create the collagen fibres. At this stage, the wound looks very tumefied and irritated.

The fibroblast number decreases, as the capillaries do, while the number of the collagen fibres increases. In this way, the granulation tissue matures to form scar tissue, a non-elastic, poorly vascularized, and poorly innervated tissue, covered by a thin, brittle skin without skin annexes.

This process, leading to the formation of a solid scar, takes about two weeks. The scar undergoes a contraction under the action of myofibrils, then a definitive settlement characterized by a compaction retraction of collagen, a colour change, and a thickness increase. These modifications may even take two years.

Wounds are a port through which foreign bodies and bacteria can penetrate the body, causing inflammations and disease states.

Therefore, it is desirable that wounds heal as soon as possible.

Wound healing can be a very time-consuming phenomenon, especially when injuries involve a large area of skin, or in the case of weakened patients, for example in the elderly, and diabetic subjects.

In order to accelerate and promote the healing of the injury, suitably formulated actives are typically applied on the wounds, such as liniments, creams, oils, and ointments, which aid and speed up the complex phenomenon of wound healing.

The commercially available preparations are undoubtedly effective, but there is still a need in the field to find alternative actives which are able to accelerate the healing of skin wounds, sores, and injuries.WO92/02218 discloses the use of the H2-receptor blocker ranitidine for the manufacture of a medicament for the topical treatment of wounds to the skin and/or to the underlying tissues, wherein ranitidine is used at a high concentration, 3-30g/1000 ml of composition.

Therefore, the object underlying the present invention is to provide a pharmaceutical preparation for topic use with wound healing activity.

It has been surprisingly found that histamine H2-receptor antagonists have a wound healing activity on wounds, burns, abrasions, and sores at particularly low concentrations.

H2-receptor antagonists are well known drugs especially in curing duodenal or gastric ulcers, for which they are typically administered in doses ranging from 150 mg to 350 mg per day. Thus they act on the H2-receptors, mainly present in the stomach and intestine.

The surprising fact underlying the present invention is that H2-receptor antagonist drugs are active in skin healing at a very low concentration, while exhibiting toxicity for higher concentrations.

This particular activity is illustrated also in the annexed Figures, in which:
Figure 1 shows the negative control comprising non-indented and non-ranitidine-treated bEnd.3 cells;
Figure 2 shows the negative control comprising indented and non-ranitidine-treated bEnd.3 cells;
Figure 3 shows indented and ranitidine-treated bEnd.3 cells at a concentration of 100 µg/mL;
Figure 4 illustrates indented and ranitidine-treated bEnd.3 cells at a concentration of 10 µg/mL;
Figure 5 shows indented and ranitidine-treated bEnd.3 cells at a concentration of 1 µg/mL.

The histamine H2-receptor antagonists advantageously used in the present invention are selected from the group consisting of ranitidine, nizatidine, and pharmaceutically acceptable salts thereof.

A pharmaceutically acceptable salt is an inorganic salt with counterions such as alkaline or earth-alkaline metal ions, such as sodium, calcium, or magnesium.

Other pharmaceutically acceptable salts are organic salts such as bismuth citrate, for example ranitidine bismuth citrate.

In a preferred aspect, the H2-receptor antagonist is ranitidine.

The above-mentioned antagonists proved to be active in healing dermatologic, vaginal, rectal, and ophthalmic wounds, sores, and injuries, at concentrations less than 100 µg/mL, preferably less than 60 µg/mL, and more preferably in amounts ranging between 20 µg/mL and 10 ng/mL.

It has been experimentally verified that antagonist concentrations equal to or higher than 100 µg/mL are toxic to epithelial cells and lead to cell death, instead of provoking a proliferation thereof, hence the healing of the wound.

Therefore, at these concentrations, the antagonist effect is precisely opposite the effect, which occurs for amounts less than 100 µg/mL.

The histamine H2-receptor antagonists are formulated, in combination with pharmacologically acceptable excipients, as a cream, spray, spray powder, dermatologic powder, gel, rectal gel, liniments, emulsions, drops for external use, ophthalmic drops, eye drops, solutions, vaginal douches, vaginal ovules, vaginal cream, rectal cream, lipstick, gauzes or patches soaked with the inventive composition.

Therefore, the H2-receptor inhibitors of the invention have a wound healing activity on epidermis, in particular, in the cure and protection of epithelial ulcerations produced whether by external agents: burnings from burns or frostbite, wounds of a most varied origin, abrasions, or by ischemic phenomena: diabetic and decubitus ulcerations.

The inhibitors of the invention also have a healing activity on vaginal, rectal and ophthalmic injuries.

In another aspect, the invention relates to a pharmaceutical composition comprising one or more of the previously-described H2-receptor antagonists in combination with a second active selected from: glycine, proline, zinc oxide, vitamin C, and vitamin E.

Glycine and proline are the simplest amino acids and play a cytoprotective role, since they provide the biological basis for the revitalization processes in metabolic depressed cells. In fact, they participate in the formation of about 60% of the collagen protein strand.

In addition, they inhibit the conductance of peripheral sensory fibres, reducing the pain symptomatology.

Vitamins C and E are fundamental in opposing the peroxide cascade which creates during the inflammatory process. The two anti-oxidants also have a strengthening action for the capillary walls, by acting on the cellular membranes. Furthermore, they stimulate the macrophages action directed to remove the debris left behind by mastocytes and basophils.

Zinc oxide is an important oligoelement in lots of enzymatic processes. Zinc reduces inner and outer injuries in the wound healing processes, speeds up the wound healing rate and increases body defences against cytological deformations.

Each of these substances, when used in the composition of the invention, contributes to enhance the healing effect of H2-receptor antagonists.

The pharmaceutical composition comprising one or more histamine H2-receptor antagonists in combination with a second active selected from: glycine, proline, zinc oxide, vitamin C, and vitamin E, is formulated, in combination with pharmacologically acceptable excipients, as a cream, spray, spray powder, dermatologic powder, gel, rectal gel, liniments, emulsions, drops for external use, ophthalmic drops, eye drops, solutions, vaginal douches, vaginal ovules, vaginal cream, rectal cream, lipstick, gauzes or patches soaked with the inventive composition.

In a further aspect, the composition of the invention comprises one or more H2-receptor antagonists, optionally in combination with a second active selected from: glycine, proline, zinc oxide, vitamin C, and vitamin E, and an antimycotic agent, preferably selected from econazole, miconazole, or itraconazole, and/or an antibacterial agent selected from erythtromycin, gentamycin, neomycin, colloidal silver, or silver sulfadiazine.

In this case, the composition can be employed to cure wounds and sores which have been infected by bacteria or mycetes, respectively.

The pharmaceutical preparations of the invention are also employed as healing agents for veterinary use.

The amounts of the various components used for the preparation of the inventive composition are set forth in the Table 1 below.

**Table 1.**

| Component | Amounts indicated as wt.% | Preferred amounts indicated as wt.% |
|---|---|---|
| Zinc oxide | 0.5-3.5 | 1-2.5 |
| Glycine | 1-6 | 2-5 |
| Proline | 1-6 | 2-5 |
| Vitamins | 0.2-3 | 0.8-1.5 |
| Antibacterial agent | 0.01-5 | 0.1-4 |
| Antifungine agent | 0.01-5 | 0.1-4 |
| Emollients, moisturising agents | q.s. | q.s. |

Preferably, when the antibacterial agent is erythromycin or colloidal silver, it will be present in the composition in amounts ranging between 0.05 and 5% by weight.

When the antibacterial agent is silver sulfadiazine, it will be preferably present in amounts ranging between 0.01 and 3% by weight.

The composition of the invention is prepared by mixing the desired components which emollient and moisturizing substances, for example vaseline and lanoline, to yield a cream texture. Alternatively, the composition can be formulated as a cream, spray, spray powder, dermatologic powder, gel, rectal gel, liniments, emulsions, drops for external use, ophthalmic drops, eye drops, solutions, vaginal douches, vaginal ovules, vaginal cream, rectal cream, lipstick, gauzes or patches soaked with the inventive composition.

### EXAMPLES

Some examples of the compositions of the invention are set forth below.

### Example 1

**Table 2. Healing cream**

| Ingredients | Amounts |
|---|---|
| | (in 100 grams) |
| ranitidine | 100 nanograms |
| glycine | 1200 mg |
| proline | 1200 mg |
| Zinc oxide | 120 mg |
| Vitamin C | 300 mg |
| Vitamin E | 120 mg |
| Excipients | q.s. |

### Example 2 (Reference example)

**Table 3. Healin cream**

| Ingredients | Amounts |
|---|---|
| | (in 100 grams) |
| Cimetidine | 100 nanograms |
| Glicine | 1200 mg |
| Proline | 1200 mg |
| Zinc oxide | 120 mg |
| Vitamin C | 300 mg |
| Vitamin E | 120 mg |
| Excipients | q.s. |

### Example 3

**Table 4. Healing and antimycotic cream.**

| Ingredients | Amounts |
|---|---|
| | (in 100 grams) |
| Ranitidine | 10 micrograms |
| Glicine | 1200 mg |
| Proline | 1200 mg |
| Zinc oxide | 120 mg |
| Econazole | 120 mg |
| Vitamin C | 300 mg |
| Vitamin E | 120 mg |
| Excipients | q.s. |

### Example 4.

**Table 5. Healin and antibacterial cream**

| Ingredients | Amounts |
|---|---|
| | (in 100 grams) |
| Ranitidine | 100 nanograms |
| Glicine | 1200 mg |
| Proline | 1200 mg |
| Zinc oxide | 120 mg |
| Gentamycin | 100 mg |
| Vitamin C | 300 mg |
| Vitamin E | 120 mg |
| Excipients | q.s. |

### Example 5.

**Table 6. Healing and antibacterial cream**

| Ingredients | Amounts |
|---|---|
| | (% by weight) |
| Ranitidine | 100 nanograms |
| Glicine | 1200 mg |
| Proline | 1200 mg |
| Neomycin | 50 mg |
| Zinc oxide | 120 mg |
| Vitamin C | 300 mg |
| Vitamin E | 120 mg |
| Excipients | q.s. |

### Example 6

**Table 7 - Healing, antibacterial and antimycotic cream**

| Ingredients | Amounts |
|---|---|
| | (% by weight) |
| Ranitidine | 100 nanograms |
| Glicine | 1200 mg |
| Proline | 1200 mg |
| Zinc oxide | 120 mg |
| Colloidal silver | 110 mg |
| Miconazole | 50 mg |
| Vitamin C | 300 mg |
| Vitamin E | 120 mg |
| Excipients | q.s. |

### Example 7

**Table 8 - Healing and antibacterial cream**

| Ingredients | Amounts |
|---|---|
| | (in 100 grams) |
| Ranitidine | 100 nanograms |
| Glicine | 1200 mg |
| Proline | 1200 mg |
| Zinc oxide | 120 mg |
| Silver sulfadiazine | 80 mg |
| Vitamin C | 300 mg |
| Vitamin E | 120 mg |
| Excipients | q.s. |

### Example 8

**Table 9 - Healing and antimycotic cream**

| Ingredients | Amounts |
|---|---|
| | (in grams) |
| Ranitidine | 100 nanograms |
| Glicine | 1200 mg |
| Proline | 1200 mg |
| Zinc oxide | 120 mg |
| Itraconazole | 105 mg |
| Vitamin C | 300 mg |
| Vitamin E | 120 mg |
| Excipients | q.s. |

### Experimental section

A ranitidine sample has been tested in order to verify the ability of healing artificial wounds induced in-vitro in human single-layer endothelial cells. This ability can be seen as a clue of a vascular reparative activity in vivo.

The cellular type which was used is represented by endothelioma-transformed cells (bEnd.3) which are universally recognised as a model mimicking the vessel wall for the in-vitro study of vessel repair and angiogenesis activity.

When cells are exposed to substances able to stimulate their ability to migrate, an essential factor in the vascular regeneration and repair process of wounds, they change their shape and modify the cytoskeleton organization, changing from stationary phenotype cells to motile phenotype cells. The bundles of microfilaments containing adhesion molecules such as vinculin, actin, and stress fiber, which keep cells anchored to the substrate, disappear, or migrate to the cellular periphery, allowing the cellular migration.

### Testing

The in-vitro experimental model which was adopted consists in cultures of murine endothelial cerebral cells, immortalized with the NTKmT retroviral vector, called bEnd.3.

### Preparation of sample

The ranitidine sample has been diluted in the culture medium and tested at the following concentrations:
100 µg/mL
10 µg/mL
1 µg/mL

### Treatment and exposure

Cells have been treated for 24 hours with the product at the end concentrations indicated in the culture medium (DMEM + 10% FCS). Cells treated only with the medium have been used as negative control. The cells are stored in an incubator at 37° C with 5% CO₂. The test has been carried out in duplicate.

### Preparation of cell cultures

The cells have been plated at 20,000 cells/well in sterile 24-well plates pre-treated with 10 µg/mL collagen and allowed growing to confluence in the previously-described culture medium, before effecting the wounds and applying samples.

### Effecting of wound and exposure of cells to the products

Once the confluence has been achieved, an artificial wound is effected throughout the single layer length with sterile plastic tip, on each sample. The cell culture medium is removed and replaced with the medium containing the tested substances, except in the negative controls.

### Evaluation of the wound repair

Immediately after the effectuation of the wound (T0), in different moments within 24 hours, new observations under phase contrast microscopy of the treated samples and the controls are made with a Leica DM-IL IMC microscope at 200/400X magnification. The images are taken with digital photographs obtained with an Olympus model C-100 camera.

### Results

### Photographic evaluation of the wound repair

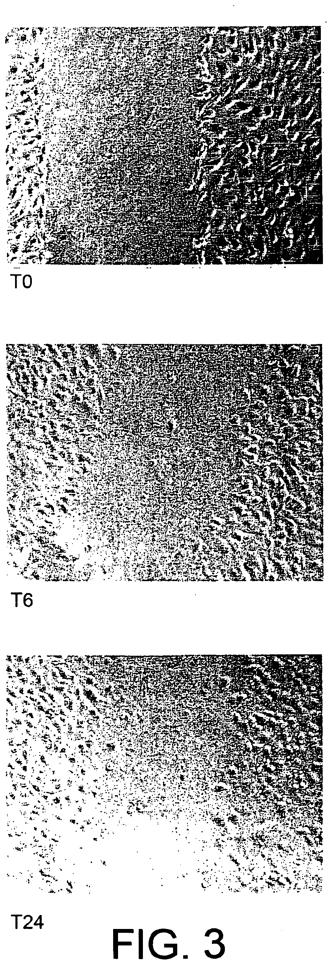

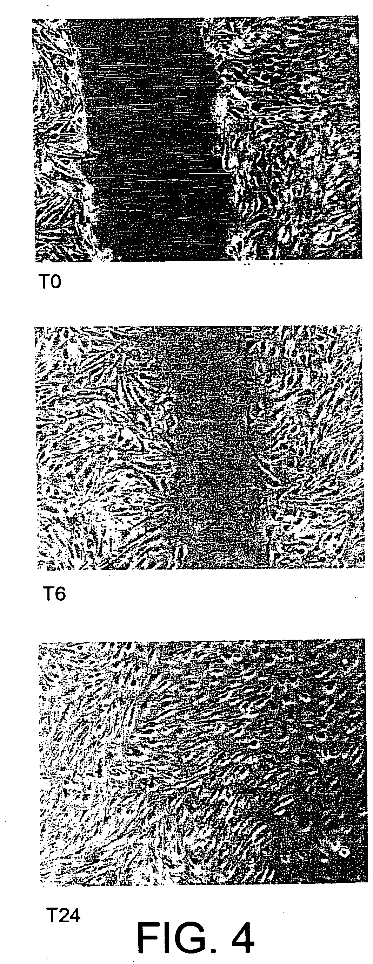

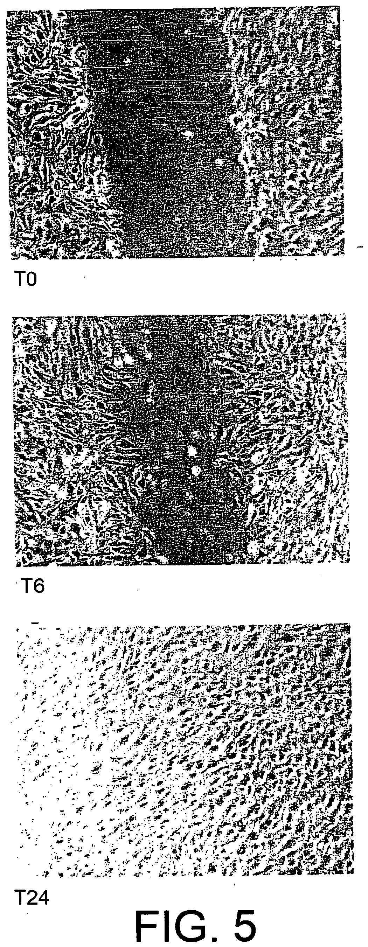

The wound repair activity has been documented over time through photographs taken with a phase-contrast optic microscope, with digital camera, obtained at time zero (T0), after six-hour treatment (T6), and after twenty-four-hour treatment (T24) (Figures 3-5).

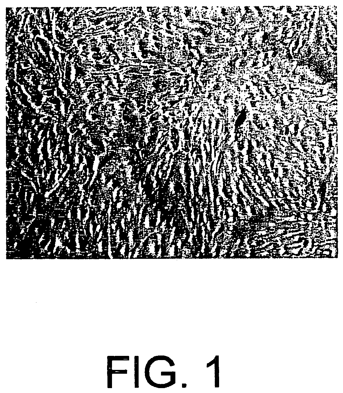

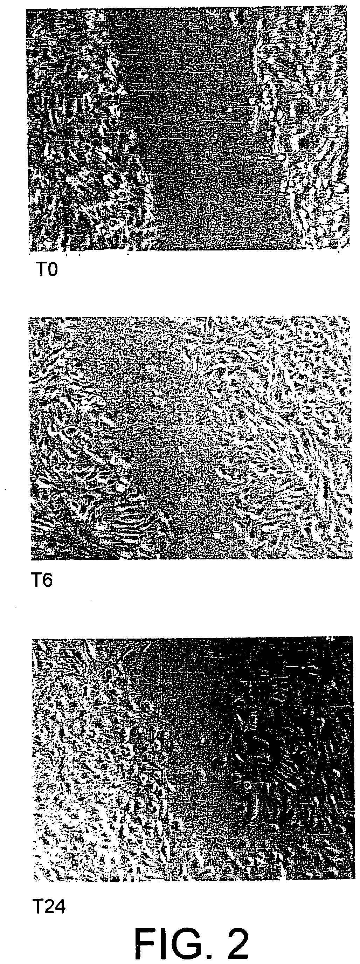

These photographs have been compared to the photographs obtained at time zero (T0), after six-hour treatment (T6), and after twenty-four-hour treatment (T24) of non-treated cell cultures used as negative control (Figures 1-2).

The above-described cell cultures have been treated with ranitidine concentrations of 100 µg/mL, 10 µg/mL, and 1 µg/mL (Figures 3-5).

A negative control, consisting in one non-indented and one indented cell cultures not treated with ranitidine, has been observed over time as a comparison (Figures 1-2).

The photographs reproduced in the Figures 3-5 show that ranitidine is cytotoxic at a 100 µg/mL concentration after 24 hours, while for concentrations <100 µg/mL, that is at 10 and 1 µg/mL, ranitidine determines a complete repair of the wound at 24 hours.

Ranitidine, at concentrations of 10 and 1 µg/mL, stimulates the migration of the endothelioma cells, causing the complete healing of the wound.

## Claims

1. A composition comprising one or more histamine H2-receptor antagonists selected from the group consisting of nizatidine, ranitidine, and pharmaceutically acceptable salts thereof at a concentration of less than 100 µg/mL for use in the topic treatment of dermatologic, vaginal, rectal, and ophthalmic wounds, abrasions, ulcerations, burns and sores.

2. The composition for use according to claim 1, wherein said one or more histamine H2-receptor antagonists is comprised in amounts less than 60 µg/mL, preferably in amounts from 20 µg/mL to 10 ng/mL.

3. The composition for use according to any claim 1 to 2, wherein said dermatologic injuries are injuries produced by external agents: burnings from burns or frostbite, wounds of a most varied origin, abrasions, or by ischemic phenomena: diabetic and decubitus ulcerations.

4. The composition for use according to any claim 1 to 3, comprising a second active selected from the group consisting of: glycine, proline, zinc oxide, vitamin C, and vitamin E.

5. The composition for use according to any claim 1 to 4 comprising an antibacterial agent.

6. The composition for use according to claim 5, wherein said antibacterial agent is selected from erythromycin, gentamycin, neomycin, colloidal silver and/or silver sulfadiazine.

7. The composition for use according to any claim 1 to 6 comprising an antimycotic agent.

8. The composition for use according to claim 7, wherein said antimycotic agent is selected from econazole, miconazole, and/or itraconazole.

9. The composition for use according to any claim 1 to 8 comprising emollient and moisturizing agents.

10. The composition for use according to claim 9, wherein said emollient and moisturizing agents are vaseline and lanoline.

11. The composition for use according to any claim 4 to 10, wherein said zinc oxide is present in amounts from 0.5% to 3.5% by weight, preferably from 1% to 2.5% by weight; said vitamin C or E are present in amounts from 0.2% to 3.0% by weight, preferably from 0.8% to 1.5% by weight; said glycine or proline are present in amounts from 1% to 6% by weight, preferably from 2% to 5% by weight.

12. The composition for use according to any claim 5 to 12, wherein said antibacterial agent is present in amounts from 0.01% to 5% by weight, preferably from 0.1% to 4% by weight, and said antimycotic agent is present in amounts from 0.01% to 5% by weight, preferably from 0.1% to 4% by weight.

13. A pharmaceutical composition for use according to any claim 1 to 12, said composition being selected from: a) ranitidine 100 ng, glycine 1200 mg, proline 1200 mg, zinc oxide 120 mg, vitamin C 300 mg, vitamin E 120 mg, excipients qs to 100 g; b) cimetidine 100 ng, glycine 1200 mg, proline 1200 mg, zinc oxide 120 mg, vitamin C 300 mg, vitamin E 120 mg, excipients qs to 100 g; c) ranitidine 10 µg, glycine 1200 mg, proline 1200 mg, zinc oxide 120 mg, econazole 120 mg, vitamin C 300 mg, vitamin E 120 mg, excipients qs to 100 g; e) ranitidine 100 ng, glycine 1200 mg, proline 1200 mg, zinc oxide 120 mg, gentamycin 100 mg, vitamin C 300 mg, vitamin E 120 mg, excipients qs to 100 g; f) ranitidine 100 ng, glycine 1200 mg, proline 1200 mg, zinc oxide 120 mg, neomycin 50 mg, vitamin C 300 mg, vitamin E 120 mg, excipients qs to 100 g; g) ranitidine 100 ng, glycine 1200 mg, proline 1200 mg, zinc oxide 120 mg, colloidal silver 110 mg, miconazole 50 mg, vitamin C 300 mg, vitamin E 120 mg, excipients qs to 100 g; h) ranitidine 100 ng, glycine 1200 mg, proline 1200 mg, zinc oxide 120 mg, vitamin C 300 mg, vitamin E 120 mg, silver sulfadiazine 80 mg, excipients qs to 100 g; i) ranitidine 100 ng, glycine 1200 mg, proline 1200 mg, zinc oxide 120 mg, vitamin C 300 mg, vitamin E 120 mg, itraconazole 105 mg, excipients qs to 100 g.

14. The composition for use according to any claim 1 to 13, wherein said composition is formulated as a form selected from cream, spray, spray powder, dermatologic powder, gel, rectal gel, liniments, emulsions, drops for external use, ophthalmic drops, eye drops, solutions, vaginal douches, vaginal ovules, vaginal cream, rectal cream, lipstick, gauzes or patches.

15. The composition for use according to any claim 5 to 14, having a healing and antibacterial, and/or healing and antimycotic effect on the epithelial and vaginal injuries.

## Patentansprüche

1. Zusammensetzung, umfassend einen oder mehrere Histamin-H2-Rezeptor-Antagonisten, ausgewählt aus der Gruppe bestehend aus Nizatidin, Ranitidin und pharmazeutisch annehmbaren Salzen davon in einer Konzentration von weniger als 100 µg/ml zur Verwendung bei der topischen Behandlung von dermatologischen, vaginalen, rektalen und ophthalmischen Wunden, Abschürfungen, Geschwüren, Verbrennungen und Entzündungen.

2. Zusammensetzung zur Verwendung nach Anspruch 1, wobei der eine oder die mehreren Histamin-H2-Rezeptor-Antagonisten in Mengen von weniger als 60 µg/mL, vorzugsweise in Mengen von 20 µg/mL bis 10 ng/mL, enthalten sind.

3. Zusammensetzung zur Verwendung nach Anspruch 1 bis 2, wobei die dermatologischen Verletzungen Verletzungen sind, die durch äußere Ursachen verursacht werden: Verbrennungen durch Verbrennungen oder Erfrierungen, Wunden unterschiedlichster Herkunft, Schürfwunden oder durch ischämische Phänomene: diabetische und Dekubitus-Geschwüre.

4. Zusammensetzung zur Verwendung nach einem der Ansprüche 1 bis 3, umfassend einen zweiten Wirkstoff, ausgewählt aus der Gruppe bestehend aus: Glycin, Prolin, Zinkoxid, Vitamin C und Vitamin E.

5. Zusammensetzung zur Verwendung nach einem der Ansprüche 1 bis 4, umfassend ein antibakterielles Mittel.

6. Zusammensetzung zur Verwendung nach Anspruch 5, wobei das antibakterielle Mittel ausgewählt ist aus Erythromycin, Gentamycin, Neomycin, kolloidalem Silber und/oder Silbersulfadiazin.

7. Zusammensetzung zur Verwendung nach einem der Ansprüche 1 bis 6, umfassend ein Antimykotikum.

8. Zusammensetzung zur Verwendung nach Anspruch 7, wobei das antimykotische Mittel ausgewählt ist aus Econazol, Miconazol und/oder Itraconazol.

9. Zusammensetzung zur Verwendung nach einem der Ansprüche 1 bis 8, umfassend erweichende und feuchtigkeitsspendende Mittel.

10. Zusammensetzung zur Verwendung nach Anspruch 9, wobei die erweichenden und feuchtigkeitsspendenden Mittel Vaseline und Lanolin sind.

11. Zusammensetzung zur Verwendung nach einem der Ansprüche 4 bis 10, wobei das Zinkoxid in Mengen von 0,5 bis 3,5 Gew.-%, vorzugsweise von 1 bis 2,5 Gew.-%, vorhanden ist; das Vitamin C oder E in Mengen von 0,2 bis 3,0 Gew.-%, vorzugsweise von 0,8 bis 1,5 Gew.-%, vorhanden sind; das Glycin oder Prolin in Mengen von 1 bis 6 Gew.-%, vorzugsweise von 2 bis 5 Gew.-%, vorhanden sind.

12. Zusammensetzung zur Verwendung nach einem der Ansprüche 5 bis 12, wobei das antibakterielle Mittel in Mengen von 0,01 bis 5 Gew.-%, vorzugsweise von 0,1 bis 4 Gew.-%, vorhanden ist und das antimykotische Mittel in Mengen von 0,01 bis 5 Gew.-%, vorzugsweise von 0,1 bis 4 Gew.-%, vorhanden ist.

13. Pharmazeutische Zusammensetzung zur Verwendung nach einem der Ansprüche 1 bis 12, wobei die Zusammensetzung ausgewählt ist aus: a) Ranitidin 100 ng, Glycin 1200 mg, Prolin 1200 mg, Zinkoxid 120 mg, Vitamin C 300 mg, Vitamin E 120 mg, Hilfsstoffe qs bis 100 g; b) Cimetidin 100 ng, Glycin 1200 mg, Prolin 1200 mg, Zinkoxid 120 mg, Vitamin C 300 mg, Vitamin E 120 mg, Hilfsstoffe qs bis 100 g; c) Ranitidin 10 µg, Glycin 1200 mg, Prolin 1200 mg, Zinkoxid 120 mg, Econazol 120 mg, Vitamin C 300 mg, Vitamin E 120 mg, Hilfsstoffe qs bis 100 g; e) Ranitidin 100 ng, Glycin 1200 mg, Prolin 1200 mg, Zinkoxid 120 mg, Gentamycin 100 mg, Vitamin C 300 mg, Vitamin E 120 mg, Hilfsstoffe qs bis 100 g; f) Ranitidin 100 ng, Glycin 1200 mg, Prolin 1200 mg, Zinkoxid 120 mg, Neomycin 50 mg, Vitamin C 300 mg, Vitamin E 120 mg, Hilfsstoffe qs bis 100 g; g) Ranitidin 100 ng, Glycin 1200 mg, Prolin 1200 mg, Zinkoxid 120 mg, kolloidales Silber 110 mg, Miconazol 50 mg, Vitamin c 300 mg, Vitamin E 120 mg, Hilfsstoffe qs bis 100 g; h) Ranitidin 100 ng, Glycin 1200 mg, Prolin 1200 mg, Zinkoxid 120 mg, Vitamin C 300 mg, Vitamin E 120 mg, Silbersulfadiazin 80 mg, Hilfsstoffe qs bis 100 g; i) Ranitidin 100 ng, Glycin 1200 mg, Prolin 1200 mg, Zinkoxid 120 mg, Vitamin C 300 mg, Vitamin E 120 mg, Itraconazol 105 mg, Hilfsstoffe qs bis 100 g.

14. Zusammensetzung zur Verwendung nach einem der Ansprüche 1 bis 13, wobei die Zusammensetzung als eine Form formuliert ist, die ausgewählt ist aus Creme, Spray, Sprühpulver, dermatologischem Puder, Gel, Rektumgel, Einreibemittel, Emulsionen, Tropfen zur äußerlichen Anwendung, ophthalmischen Tropfen, Augentropfen, Lösungen, Vaginalduschen, Vaginalovula, Vaginalcreme, Rektumcreme, Lippenstift, Gaze oder Pflaster.

15. Zusammensetzung zur Verwendung nach einem der Ansprüche 5 bis 14, die eine heilende und antibakterielle und/oder heilende und antimykotische Wirkung auf die epithelialen und vaginalen Verletzungen aufweist.

## Revendications

1. Composition comprenant un ou plusieurs antagonistes de récepteur H2 d'histamine sélectionnés dans le groupe consistant en nizatidine, ranitidine et des sels pharmaceutiquement acceptables de ceux-ci en une concentration inférieure à 100 µg/mL pour une utilisation dans le traitement topique de plaies dermatologiques, vaginales, rectales et ophtalmiques, des abrasions, ulcérations, brûlures et érosions.

2. Composition pour une utilisation selon la revendication 1, dans laquelle lesdits un ou plusieurs antagonistes de récepteur H2 d'histamine sont compris en quantités inférieures à 60 µg/mL, de préférence en quantités de 20 µg/mL à 10 ng/mL.

3. Composition pour une utilisation selon l'une quelconque des revendications 1 à 2, dans laquelle lesdites lésions dermatologiques sont des lésions produites par des agents externes : brûlures dues à un coup de soleil ou des engelures, plaies d'origines les plus diverses, abrasions ou dues à un phénomène ischémique : ulcérations diabétiques et escarres de décubitus.

4. Composition pour une utilisation selon l'une quelconque des revendications 1 à 3, comprenant un second principe actif sélectionné dans le groupe constitué de : glycine, proline, oxyde de zinc, vitamine C et vitamine E.

5. Composition pour une utilisation selon l'une quelconque des revendications 1 à 4, comprenant un agent antibactérien.

6. Composition pour une utilisation selon la revendication 5, dans laquelle ledit agent antibactérien est sélectionné parmi l'érythromycine, la gentamycine, la néomycine, l'argent colloïdal et/ou la sulfadiazine d'argent.

7. Composition pour une utilisation selon l'une quelconque des revendications 1 à 6, comprenant un agent antimycosique.

8. Composition pour une utilisation selon la revendication 7, dans laquelle ledit agent antimycosique est sélectionné parmi l'éconazole, le miconazole et/ou l'itraconazole.

9. Composition pour une utilisation selon l'une quelconque des revendications 1 à 8, comprenant des agents émollients et hydratants.

10. Composition pour une utilisation selon la revendication 9, dans laquelle lesdits agents émollients et hydratants sont la vaseline et la lanoline.

11. Composition pour une utilisation selon l'une quelconque des revendications 4 à 10, dans laquelle ledit oxyde de zinc est présent en quantités de 0,5 % à 3,5 % en poids, de préférence de 1 % à 2,5 % en poids ; lesdites vitamines C ou E sont présentes en quantités de 0,2 % à 3,0 % en poids, de préférence de 0,8 % à 1,5 % en poids ; lesdites glycine ou proline sont présentes en quantité de 1 % à 6 % en poids, de préférence de 2 % à 5 % en poids.

12. Composition pour une utilisation selon l'une quelconque des revendications 5 à 12, dans laquelle ledit agent antibactérien est présent en quantités de 0,01 % à 5 % en poids, de préférence de 0,1 % à 4 % en poids, et ledit agent antimycosique est présent en quantités de 0,01 % à 5 % en poids, de préférence de 0,1 % à 4 % en poids.

13. Composition pharmaceutique pour une utilisation selon l'une quelconque des revendications 1 à 12, ladite composition étant sélectionnée parmi: a) ranitidine 100 ng, glycine 1 200 mg, proline 1 200 mg, oxyde de zinc 120 mg, vitamine C 300 mg, vitamine E 120 mg, excipients en quantité suffisante pour compléter à 100 g ; b) cimétidine 100 ng, glycine 1 200 mg, proline 1 200 mg, oxyde de zinc 120 mg, vitamine C 300 mg, vitamine E 120 mg, excipients en quantité suffisante pour compléter à 100 g ; c) ranitidine 10 µg, glycine 1 200 mg, proline 1 200 mg, oxyde de zinc 120 mg, éconazole 120 mg, vitamine C 300 mg, vitamine E 120 mg, excipients en quantité suffisante pour compléter à 100 g ; e) ranitidine 100 ng, glycine 1 200 mg, proline 1 200 mg, oxyde de zinc 120 mg, gentamycine 100 mg, vitamine C 300 mg, vitamine E 120 mg, excipients en quantité suffisante pour compléter à 100 g ; f) ranitidine 100 ng, glycine 1 200 mg, proline 1 200 mg, oxyde de zinc 120 mg, néomycine 50 mg, vitamine C 300 mg, vitamine E 120 mg, excipients en quantité suffisante pour compléter à 100 g; g) ranitidine 100 ng, glycine 1 200 mg, proline 1 200 mg, oxyde de zinc 120 mg, argent colloïdal 110 mg, miconazole 50 mg, vitamine C 300 mg, vitamine E 120 mg, excipients en quantité suffisante pour compléter à 100 g; h) ranitidine 100 ng, glycine 1 200 mg, proline 1 200 mg, oxyde de zinc 120 mg, vitamine C 300 mg, vitamine E 120 mg, sulfadiazine d'argent 80 mg, excipients en quantité suffisante pour compléter à 100 g; i) ranitidine 100 ng, glycine 1 200 mg, proline 1 200 mg, oxyde de zinc 120 mg, vitamine C 300 mg, vitamine E 120 mg, itraconazole 105 mg, excipients en quantité suffisante pour compléter à 100 g.

14. Composition pour une utilisation selon l'une quelconque des revendications 1 à 13, dans laquelle ladite composition est préparée sous une forme sélectionnée parmi une crème, un spray, une poudre pour spray, une poudre dermatologique, un gel, un gel rectal, des onguents, des émulsions, des gouttes pour une utilisation externe, des gouttes ophtalmiques, des gouttes oculaires, des solutions, des douches vaginales, des ovules vaginaux, une crème vaginale, une crème rectale, un rouge à lèvres, des gazes ou des patchs.

15. Composition pour une utilisation selon l'une quelconque des revendications 5 à 14, ayant un effet cicatrisant et antibactérien et/ou un effet cicatrisant et antimycosique sur les lésions épithéliales et vaginales.
